# FASCICULE DE BREVET EUROPEEN

(11) **EP 1 221 435 B1**
(45) Date de publication et mention de la délivrance du brevet: **14.04.2004**
(21) Numéro de dépôt: 01403230.4
(22) Date de dépôt: 13.12.2001
(51) Int. Cl.: C07C 29/82, B01D 3/36, C10L 3/10

(54) **Procédé de récupération du méthanol contenu dans une charge d'hydrocarbures liquides**
Verfahren zur Gewinnung von Methanol aus einer flüssigen Kohlenwasserstoffbeschickung
Process for the recovery of methanol from a liquid hydrocarbon feedstock

(30) Priorité: 09.01.2001 FR 0100283
(43) Date de publication de la demande: 10.07.2002
(73) Titulaire: Institut Français du Pétrole, 92852 Rueil-Malmaison Cedex (FR)
(72) Inventeur: Laborie, Géraldine, 92400 Courbevoie (FR); Lecomte, Fabrice, 92500 Rueil Malmaison (FR); Pucci, Annick, 78290 Croissy sur Seine (FR)

(56) Documents cités:
- FR-A- 2 605 241
- FR-A- 2 636 857
- US-A- 4 014 667

## Description

La présente invention a pour objet un procédé de récupération du méthanol contenu dans une charge d'hydrocarbures liquides.

Les brevets FR-B-2.605.241 et FR-B-2.636.857 proposent un traitement du gaz naturel qui permet au moins de réaliser la déshydratation et la récupération des liquides de gaz naturel contenus dans le gaz par abaissement de la température. Le gaz est protégé contre la formation des hydrates par ajout de méthanol. A l'issue de l'étape de refroidissement, trois phases sont séparées dans un ballon de décantation (une phase liquide aqueuse, une phase liquide d'hydrocarbures appelée les « liquides de gaz naturel » ou « LGN » et une phase gazeuse constituée de méthane), ces trois phases contenant toutes du méthanol.

Pour des raisons économiques, il est intéressant de récupérer le méthanol contenu dans la phase liquide d'hydrocarbures.

Dans le brevet US-A-4.014.667, il est décrit un procédé de récupération du méthanol présent dans une phase liquide d'hydrocarbures. Ce procédé est un lavage à l'eau de la phase liquide d'hydrocarbures et se fait dans un appareil de contactage liquide/liquide, par exemple un mélangeur/contacteur. Après mis en contact, la phase liquide d'hydrocarbures est débarrassée presque en totalité du méthanol et subit ensuite une opération de séparation des hydrocarbures la constituant. L'opération de séparation peut être effectuée dans une colonne de distillation située en aval de l'appareil de contactage liquide/liquide.

Ce procédé de lavage à l'eau présente les avantages d'être relativement simple, de ne pas nécessiter de coûts d'installation et de fonctionnement importants, et de permettre de récupérer une fraction importante du méthanol présent dans la phase liquide d'hydrocarbures.

Néanmoins, le procédé selon le brevet US-A-4.014.667 présente certains inconvénients. A la sortie du mélangeur/décanteur, la phase liquide d'hydrocarbures est saturée en eau et doit passer par une étape supplémentaire de déshydratation.

En outre, le contactage liquide/liquide de la phase liquide d'hydrocarbures avec de l'eau, situé en aval de la colonne de distillation, a au moins deux désavantages. D'une part, le contactage peut provoquer un refroidissement de la phase hydrocarbure avant l'entrée dans la colonne de distillation, ce qui nécessite un apport d'énergie plus important dans la colonne de distillation, pour opérer la séparation des hydrocarbures. D'autre part, lors du contactage, la phase hydrocarbure se charge en eau, ce qui augmente la quantité de matière à traiter dans la colonne de distillation.

De plus, ce procédé de lavage à l'eau ne permet de récupérer qu'une solution aqueuse faiblement concentrée en méthanol. Il est alors nécessaire d'utiliser une colonne de fractionnement pour séparer le méthanol de l'eau de lavage.

L'invention propose un nouveau procédé de récupération du méthanol contenu dans une phase liquide d'hydrocarbures. Ce procédé peut s'appliquer au traitement des liquides de gaz naturel obtenus en sortie des procédés décrits dans les brevets FR-B-2.605.241 et FR-B-2.636.857. Ce nouveau procédé permet de remédier aux inconvénients précités.

La présente invention utilise le fait que le méthanol forme des azéotropes avec le propane et le butane. Les azéotropes méthanol-propane et méthanol-butane ont une température d'ébullition inférieure à celle des corps purs propane et butane et substantiellement différente des autres alcanes. Ainsi, il est possible de séparer par distillation les azéotropes de méthanol des autres hydrocarbures et notamment du propane et du butane. De ce fait, on peut isoler le méthanol dans un faible flux de matière par rapport au flux à traiter.

La présente invention propose d'isoler les azéotropes formés dans une colonne de distillation par le méthanol, le propane et le butane. Les azéotropes sont ensuite liquéfiés et mélangés avec de l'eau afin de dissoudre le méthanol dans l'eau. Puis le mélange est introduit dans un bac de décantation pour séparer la phase aqueuse de la phase hydrocarbure liquide. Et enfin, on soutire une phase aqueuse contenant du méthanol et on recycle la phase hydrocarbure appauvrie en méthanol vers la colonne de distillation à titre de reflux.

Le procédé selon l'invention est défini de façon générale comme un procédé de récupération du méthanol contenu dans une charge liquide d'hydrocarbures, comportant les étapes suivantes :
a) on distille la phase liquide d'hydrocarbures contenant le méthanol pour isoler une phase gazeuse constituée d'azéotropes méthanol-hydrocarbures,
b) on mélange ladite phase gazeuse obtenue à l'étape a) avec de l'eau,
c) on sépare le mélange obtenu à l'étape b) en une phase aqueuse riche en méthanol et en une phase hydrocarbure pauvre en méthanol, ladite phase hydrocarbure étant recyclée à l'étape a).

Selon le procédé de l'invention, les azéotropes isolés peuvent être du méthanol-propane et du méthanol-butane. Soit avant l'étape b), on peut liquéfier ladite phase gazeuse obtenue à l'étape a), soit à l'étape b), ladite phase gazeuse peut être liquéfiée lors du mélange avec l'eau. A l'étape c), on peut séparer le mélange obtenu à l'étape b) par décantation. Avant l'étape a), on peut prélever par distillation le méthane et l'éthane de la phase liquide d'hydrocarbures: A l'étape a), on peut obtenir une phase liquide contenant les hydrocarbures constitués de plus de cinq atomes de carbone et une phase gazeuse contenant du propane et du butane.

On peut utiliser le procédé selon l'invention pour traiter la phase liquide d'hydrocarbure contenant du méthanol provenant d'un procédé de déshydratation ou d'un procédé de déshydratation et de désacidification d'un gaz naturel brut.

On peut utiliser le procédé selon l'invention, dans lequel la phase aqueuse riche en méthanol obtenue à l'étape c), est recyclée dans un procédé de déshydratation ou de déshydratation et de désacidification d'un gaz naturel brut.

Le procédé selon l'invention offre de nombreux avantages par rapport à l'art antérieur.

Le procédé selon l'invention permet de réaliser des économies d'énergie. N'ayant pas de lavage à l'eau situé en amont de la colonne de séparation des hydrocarbures, la phase liquide d'hydrocarbures est ni refroidie, ni saturée en eau. Ainsi, la colonne de distillation traite un flux de moindre débit et plus chaud, et de ce fait, nécessite moins de chaleur pour effectuer la séparation des hydrocarbures et des azéotropes. De plus, selon le procédé de l'invention, le mélange des azéotropes obtenu en tête de la colonne de distillation avec de l'eau permet de réduire la température desdits azéotropes, voire de les liquéfier.

En outre, le procédé selon l'invention nécessite un flux d'eau moins important que le procédé par lavage selon l'art antérieur. Selon l'invention, ce flux d'eau n'a pas obligation de dissoudre tout le méthanol contenu dans le mélange azéotropique. En effet, la phase liquide d'hydrocarbures obtenue après le mélange avec l'eau est recyclée dans le procédé à titre de reflux dans la colonne de distillation. Ainsi, on peut atteindre une concentration de 10% molaire de méthanol dans la phase aqueuse soutirée.

L'invention sera mieux comprise à la lecture de la description suivante, faite à titre illustratif et nullement limitatif, en référence aux figures annexées parmi lesquelles :
- la figure 1 représente de façon schématique le procédé de l'invention intégré à un procédé de déshydratation et de dégazolinage de gaz naturel,
- la figure 2 schématise un mode de réalisation du procédé selon l'invention.

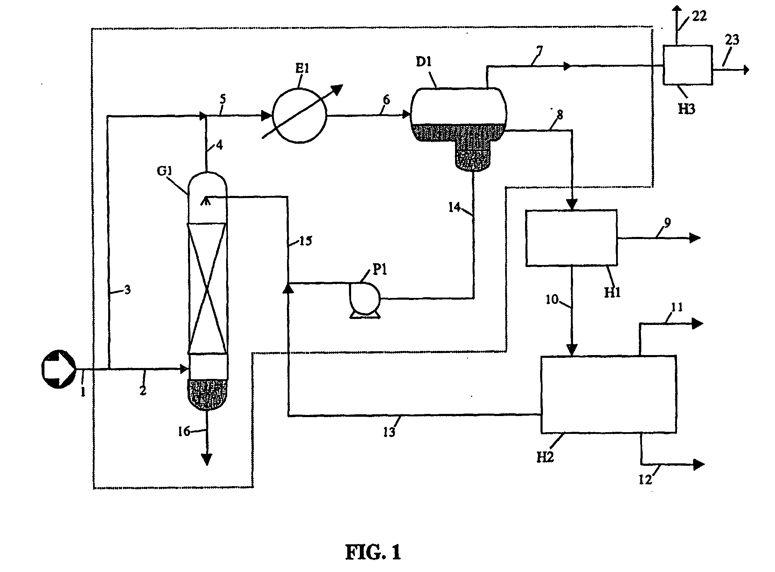

Sur la figure 1, le procédé de récupération du méthanol selon l'invention schématisé par les symboles H1 et H2 est placé à la suite d'un procédé de déshydratation et de dégazolinage tel que décrit dans le brevet FR-B-2.605.241. Ce procédé de déshydratation et de dégazolinage est schématisé sur la figure 1 par les symboles contenus dans le cadre en pointillés. Le procédé décrit dans le brevet FR-B-2.636.857 reprend le procédé du brevet FR-B-2.605.241 et y ajoute une étape de désacidification représentée sur la figure 1 par le symbole H3.

En référence à la figure 1, le gaz naturel brut à traiter arrive par le conduit 1. Une fraction du gaz à traiter est introduite, par l'intermédiaire du conduit 2, dans la zone de contact G1 formée par exemple d'un garnissage. Le gaz est mis en contact à contre courant avec une phase liquide formée de méthanol et d'eau introduite par le conduit 15 dans la zone de contact G1.

Le gaz naturel sortant en tête de la zone de contact G1 renferme de la vapeur d'eau et du méthanol, le plus souvent en quantités voisines de la saturation. Ce gaz est recueilli dans le conduit 4 et mélangé à la fraction restante de gaz naturel brut provenant du conduit 1 par le conduit 3. Une fraction aqueuse décante au fond de la zone de contact G1 et est évacuée par le conduit 16.

Le mélange, acheminé par le conduit 5, est ensuite refroidi dans un échangeur de chaleur E1 par un fluide frigorigène. Le refroidissement opéré dans l'échangeur E1 permet de condenser une fraction des hydrocarbures de masse moléculaire supérieure ou égale à l'éthane. Il permet également de condenser la majeure partie de l'eau et du méthanol qui étaient contenus dans le gaz arrivant dans l'échangeur E1.

Le fluide constitué de condensat et de gaz en sortie de l'échangeur E1 est envoyé dans une zone de décantation D1 par l'intermédiaire du conduit 6. Dans la zone de décantation D1, trois phases sont séparées : une phase aqueuse contenant du méthanol, une phase liquide d'hydrocarbures correspondant aux alcanes de masse moléculaire supérieure ou égale à l'éthane et une phase gazeuse correspondant au méthane du gaz naturel déshydraté. Le méthane déshydraté est évacué de la zone de décantation D1 par le conduit 7. La phase aqueuse contenant du méthanol est quant à elle soutirée par la conduite 14 puis envoyée au moyen de la pompe P1 et du conduit 15 à la zone de contact G1.

Selon le procédé décrit dans le brevet FR-B-2.636.857, le méthane déshydraté est envoyé par le conduit 7 vers un dispositif H3 de désacidification. A l'issue du procédé de désacidification mis en oeuvre dans le dispositif H3, un flux gazeux constitué de méthane déshydraté et débarrassé des gaz acides sort par le conduit 22 tandis que les gaz acides sont évacués par le conduit 23.

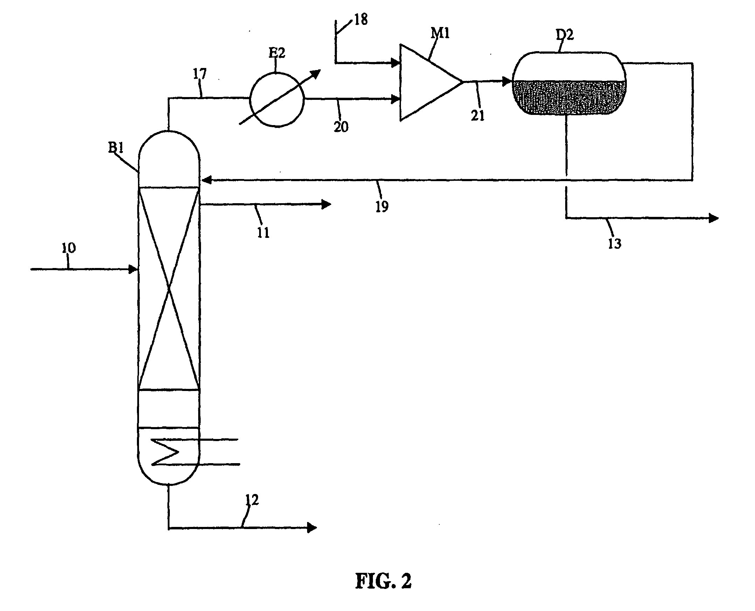

La phase liquide d'hydrocarbures séparée dans la zone de décantation D1 constitue la charge de matière à traiter par le procédé selon la présente invention. Le procédé est représenté sur la figure 1 par les symboles H1 et H2. La figure 2 détaille le procédé représenté par le symbole H2 à la figure 1.

La phase liquide d'hydrocarbures est envoyée par le conduit 8 dans un dééthaniseur H1 afin d'éliminer le méthane et l'éthane encore présents dans la phase liquide. Le dééthaniseur H1 peut être constitué d'une colonne de distillation connue de l'homme du métier. Le méthane et l'éthane sont évacués par le conduit 9.

En référence à la figure 2, la phase liquide d'hydrocarbures à traiter, récupérée en sortie du dééthaniseur H1 par le conduit 10, renferme du propane, du butane, de l'i-butane, du n-butane, du i-pentane, du n-pentane, de l'hexane et de l'heptane ainsi que du méthanol. Elle est envoyée par le conduit 10 dans une colonne de distillation B1 afin de fractionner et de récupérer une phase gazeuse d'azéotropes méthanol-propane et méthanol-butane formés dans ladite colonne B1, une coupe gazeuse propane/butane et une coupe liquide d'hydrocarbures, constitués majoritairement d'au moins cinq atomes de carbone, couramment appelée gazoline. La colonne B1 reçoit à titre de reflux une phase liquide constituée de propane, de butane et de méthanol par le conduit 19.

En fond de la colonne B1, on soutire par le conduit 12 une phase liquide d'hydrocarbures correspondant à la gazoline. Au niveau intermédiaire, entre le fond et la tête de la colonne, présentant les conditions thermodynamiques adéquates, on soutire par le conduit 11 la coupe propane/butane pure. On évacue, en tête de la colonne B1 par le conduit 17, une phase gazeuse contenant les azéotropes méthanol-propane et méthanol-butane formés lors de la distillation.

La phase gazeuse récupérée en tête de la colonne B1 par le conduit 17 est refroidie dans un échangeur de chaleur E2 afin de provoquer une condensation complète des azéotropes. La phase liquide, issue de l'échangeur E2 par le conduit 20, est mise en contact dans la zone de contact M1 avec un courant d'eau arrivant par le conduit 18. Le mélange effectué dans la zone de contact M1 est envoyé dans le bac de décantation D2 par le conduit 21.

L'opération de condensation dans l'échangeur de chaleur E2 n'est pas nécessaire si le débit d'eau arrivant dans la zone de contact M1 est suffisamment froid et important pour liquéfier les azéotropes par simple mise en contact gaz/liquide.

Deux phases liquides se séparent dans le bac de décantation D2, une phase d'hydrocarbures contenant du propane, du butane et une faible fraction de méthanol et une phase aqueuse riche en méthanol. La phase aqueuse est soutirée du dispositif par l'intermédiaire du conduit 13. Cette phase aqueuse peut être recyclée dans le procédé selon le brevet FR-B-2.605.241 ou le brevet FR-B-2.636.857 par les conduits 13 et 15 vers la zone de contact G1. La phase d'hydrocarbures est, quant à elle, recyclée en tête de la colonne de distillation B1 par l'intermédiaire du conduit 19, à titre de reflux.

La suite de la description propose un exemple de mise en oeuvre du procédé selon l'invention décrite en liaison avec les figures 1 et 2. Cet exemple donné à titre d'illustration ne limite en rien la portée de l'invention.

On traite une charge liquide d'hydrocarbures en sortie d'un dééthaniseur H1 en aval du procédé de déshydratation et de dégazolinage réalisé selon le brevet FR-B-2.605.241 ou brevet FR-B-2.636.857 qui contient 48,6% molaire de propane, 14,9% de n-butane, 13,9% de i-butane, 5,6% de n-pentane, 5,6% de i-pentane, 4,1% d'hexane, 6,3% d'heptane et 0,9% de méthanol. La charge provient du pied du dééthaniseur par le conduit 10 et présente une température de 75 °C et une pression de 17 bar. Le débit de la charge est égal à 1824,64 kmol/h.

Après avoir été comprimée à 20 bar, la charge est introduite dans la colonne B 1 afin d'être fractionnée en une coupe propane/butane et en une coupe contenant les hydrocarbures constitués majoritairement de plus de cinq atomes de carbone. La colonne B 1 est une colonne de distillation comportant 40 plateaux théoriques et fonctionnant sous une pression de 20 bar. L'alimentation de la colonne par le conduit 10 se fait au niveau du vingtième plateau. Dans la colonne B1, les azéotropes méthanol-propane et méthanol-butane se forment et montent en tête de la colonne de distillation.

La phase gazeuse constituée des azéotropes sort de la colonne B1 par le conduit 17, elle est refroidie dans l'échangeur E2 jusqu'à une température de 62 °C, ce qui provoque une condensation totale. Le flux de liquide récupéré à la sortie du condenseur E2 est mélangé à de l'eau dans un mélangeur M1 afin de dissoudre le méthanol dans l'eau. Le mélangeur M1 peut être un appareil commercialisé par la société Sulzer. Le débit d'eau introduit au niveau du conduit 18 est égal à 75 kmol/h.

Le mélange constitué est envoyé dans le séparateur D2 où se séparent deux phases liquides : une phase aqueuse contenant une fraction de méthanol et une phase d'hydrocarbures contenant des traces de méthanol. La phase aqueuse est soutirée du système par l'intermédiaire du conduit 13, son débit est égal à 82,9 kmol/h et elle contient 9,5% molaire de méthanol. La phase aqueuse est recyclée dans le procédé de déshydratation et de dégazolinage selon le brevet FR-B-2605241 ou le brevet FR-B-2.636.857 (voir. figure 1) en tête de la colonne G1. La quantité totale de méthanol récupéré est égale à 50 %. La phase hydrocarbure est, quant à elle, retournée à la colonne de distillation B1 par l'intermédiaire de la conduite 19. Le débit de ce flux est égal à 2822 kmol/h.

La coupe propane/butane est soutirée au niveau du deuxième plateau de la colonne B 1 grâce à la conduite 11. Le débit du soutirage est égal à 1415 kmol/h de façon à avoir un taux de reflux égal à 2 au niveau de la colonne B1. Ce taux de reflux est un compromis entre la quantité totale de méthanol récupéré et la concentration du méthanol dans la phase aqueuse soutirée par le conduit 15. La coupe contient 62,7 % de propane, 17,9 % de i-butane, 18,8 % de n-butane.

La coupe correspondant aux hydrocarbures plus lourds, comprenant majoritairement au moins cinq atomes de carbone, est soutirée en fond de la colonne B1 par l'intermédiaire du conduit 12.

## Revendications

1. Procédé de récupération du méthanol contenu dans une charge liquide d'hydrocarbures, comportant les étapes suivantes :
a) on distille la phase liquide d'hydrocarbures contenant le méthanol pour isoler une phase gazeuse constituée d'azéotropes méthanol-hydrocarbures,
b) on mélange ladite phase gazeuse obtenue à l'étape a) avec de l'eau,
c) on sépare le mélange obtenu à l'étape b) en une phase aqueuse riche en méthanol et en une phase hydrocarbure pauvre en méthanol, ladite phase hydrocarbure étant recyclée à l'étape a).

2. Procédé selon la revendication 1) dans lequel à l'étape a), les azéotropes isolés sont du méthanol-propane et du méthanol-butane.

3. Procédé selon l'une des revendications 1 et 2 dans lequel avant l'étape b), on liquéfie ladite phase gazeuse obtenue à l'étape a).

4. Procédé selon l'une des revendications 1 et 2 dans lequel à l'étape b), ladite phase gazeuse est liquéfiée lors du mélange avec l'eau.

5. Procédé selon l'une des revendications 3 et 4 dans lequel à l'étape c), on sépare le mélange obtenu à l'étape b) par décantation.

6. Procédé selon l'une des revendications précédentes dans lequel avant l'étape a) on prélève par distillation le méthane et l'éthane de la phase liquide d'hydrocarbures.

7. Procédé selon la revendication 6 dans lequel à l'étape a) on obtient une phase liquide contenant les hydrocarbures constitués de plus de cinq atomes de carbone et une phase gazeuse contenant du propane et du butane.

8. Procédé selon l'une des revendications 1 à 7 dans laquelle la phase liquide d'hydrocarbure contenant du méthanol provient d'un procédé de déshydratation d'un gaz naturel brut.

9. Procédé selon l'une des revendications 1 à 7 dans laquelle la phase liquide d'hydrocarbure contenant du méthanol provient d'un procédé de déshydratation et de désacidification d'un gaz naturel brut.

10. Procédé selon l'une des revendications 8 et 9 dans laquelle la phase aqueuse riche en méthanol, obtenue à l'étape c) du procédé de récupération du méthanol dans une phase liquide d'hydrocarbures, est recyclée dans le procédé de déshydratation ou de déshydratation et de désacidification d'un gaz naturel brut.

## Patentansprüche

1. Verfahren zur (Rück)gewinnung des in einer flüssigen Kohlenwasserstoffcharge enthaltenen Methanols, die folgenden Stufen umfassend:
a. man destilliert die flüssige das Methanol enthaltende Kohlenwasserstoffcharge, um eine gasförmige Phase zu isolieren, die aus Methanol-Kohlenwasserstoffen-Azeotropen gebildet ist,
b. man mischt diese gasförmige in Stufe a) erhaltene Phase mit Wasser und
c. man trennt das in Stufe b) erhaltene Gemisch in eine wässrige an Methanol reiche Phase und eine an Methanol verarmte Kohlenwasserstoffphase, wobei die Kohlenwasserstoffphase in die Stufe a) rezykliert wird.

2. Verfahren nach Anspruch 1, bei dem in Stufe a) die isolierten Azeotrope Methanol-Propan und Methanol-Butan sind.

3. Verfahren nach Anspruch 1 und 2, bei dem vor der Stufe b) man diese gasförmige in Stufe a) erhaltene Phase verflüssigt.

4. Verfahren nach einem der Ansprüche 1 und 2, bei dem in Stufe a) diese gasförmige Phase während des Mischens mit Wasser verflüssigt wird.

5. Verfahren nach einem der Ansprüche 3 und 4, bei dem in Stufe c) man das in Stufe b) erhaltene Gemisch durch Dekantierung abtrennt.

6. Verfahren nach einem der vorhergehenden Ansprüche, bei dem vor der Stufe a) man durch Destillation das Methan und Ethan aus der flüssigen Kohlenwasserstoffcharge entnimmt.

7. Verfahren nach Anspruch 6, bei dem in Stufe a) man eine flüssige Phase, die Kohlenwasserstoffe enthält, die aus mehr als fünf Kohlenstoffatomen gebildet sind sowie eine gasförmige Propan und Butan enthaltene Phase, erhält.

8. Verfahren nach einem der Ansprüche 1 bis 7, bei dem die flüssige Methanol enthaltende Kohlenwasserstoffcharge aus einem Verfahren der Dehydratation eines rohen Erdgases stammt.

9. Verfahren nach einem der Ansprüche 1 bis 7, bei dem die flüssige Methanol enthaltende Kohlenwasserstoffcharge aus einem Verfahren der Dehydratation und Entsäuerung eines rohen Erdgases stammt.

10. Verfahren nach einem der Ansprüche 8 und 9, bei dem die an Methanol reiche wässrige Phase, die in Stufe c) des Verfahrens zur Rückgewinnung des Methanols in einer flüssigen Kohlenwasserstoffcharge erhalten wurde, in das Verfahren der Dehydratation oder Dehydratation und Entsäuerung eines rohen Erdgases rezykliert wird.

## Claims

1. A process for recovering methanol contained in a liquid hydrocarbon feed, comprising the following stages :
a) distilling the liquid hydrocarbon phase containing the methanol so as to isolate a gas phase consisting of methanol-hydrocarbon azeotropes,
b) mixing said gas phase obtained in stage a) with water,
c) separating the mixture obtained in stage b) into a methanol-rich aqueous phase and a methanol-poor hydrocarbon phase, said hydrocarbon phase being recycled to stage a).

2. A process as claimed in claim 1 wherein, in stage a), the azeotropes isolated are methanol-propane and methanol-butane.

3. A process as claimed in any one of claims 1 and 2 wherein, before stage b), said gas phase obtained in stage a) is liquefied.

4. A process as claimed in any one of claims I and 2 wherein, in stage b), said gas phase is liquefied during mixing with the water.

5. A process as claimed in any one of claims 3 and 4 wherein, in stage c), the mixture obtained in stage b) is separated by decantation.

6. A process as claimed in any one of the previous claims wherein, before stage a), the methane and the ethane are separated from the liquid hydrocarbon phase by distillation.

7. A process as claimed in claim 6 wherein, in stage a), a liquid phase containing the hydrocarbons consisting of more than five carbon atoms and a gas phase containing propane and butane are obtained.

8. A process as claimed in any one of claims 1 to 7, wherein the liquid hydrocarbon phase containing methanol comes from a raw natural gas dehydration process.

9. A process as claimed in any one of claims 1 to 7, wherein the liquid hydrocarbon phase containing methanol comes from a raw natural gas dehydration and deacidizing process.

10. A process as claimed in any one of claims 8 and 9, wherein the methanol-rich aqueous phase obtained in stage c) of the process for recovering the methanol present in a liquid hydrocarbon phase is recycled to the raw natural gas dehydration or dehydration and deacidizing process.
